# EUROPEAN PATENT SPECIFICATION

(11) **EP 0 358 468 B1**
(45) Date of publication and mention of the grant of the patent: **16.03.1994**
(21) Application number: 89309002.7
(22) Date of filing: 06.09.1989
(51) Int. Cl.: C12P 21/08, G01N 33/68, C12P 21/00

(54) **Monoclonal antibody to high molecular weight human epidermal growth factor**
Monoklonale Antikörper gegen den menschlichen Epidermal-Wachstumsfaktor mit hohem Molekulargewicht
Anticorps monoclonal contre le facteur de croissance de l'épiderme humain de poids moléculaire élevé

(30) Priority: 06.09.1988 JP 223053/88
(43) Date of publication of application: 14.03.1990
(73) Proprietor: NIHON CHEMICAL RESEARCH KABUSHIKI KAISHA also known as JCR PHARMACEUTICALS CO., LTD, Kobe Hyogo 658 (JP)
(72) Inventor: Koga, Junichi, Nishi-ku Kobe Hyogo 673 (JP); Nishimuro, Satoshi, Higashinada-ku Kobe Hyogo 658 (JP); Hiratani, Hajime, Sennan-gun Osaka 599-02 (JP)
(74) Representative: Burford, Anthony Frederick

(56) References cited:
- CHEMICAL ABSTRACTS, vol. 111, no. 17, 23 October 1989, Columbus, OH (US); J. KOGA et al., p. 77, no. 146914h#

## Description

The present invention relates to a monoclonal antibody to a high-molecular-weight human epidermal growth factor, a method of producing the same and a method of using the same.

Human epidermal growth factor is known to act as a mitogenic factor in vitro. Its actions in living organisms, for example tissue differentiation promoting action and promoter action on various types of tumor, have been demonstrated as well. Clinically, as is well known, the possibility of its use in ophthalmic preparations for the treatment of keratitis, corneal ulcer and the like or as a therapeutic agent for gastric ulcer, burn injury and the like is under investigation.

Recently, the presence of a precursor to human epidermal growth factor (hereinafter referred to as "hEGF" for short) has been predicted from the mRNA side [Alane Grey et al., Nature, 303, 722-725 (1983); Robert J. Rall et al., Nature, 313, 228-231 (1985)]. Another group of researchers has demonstrated that a substance which immunologically crosses hEGF and competes therewith for the receptor is present in urine [Charles D. Mount. et al., Archives of Biochemistry and Biophysics, 255, 1-7 (1987)]. This substance (hereinafter referred to as "HMW-hEGF" for short) is an O-glycosylated polypeptide having a molecular weight of about 33,000 daltons while hEGF has a molecular weight of about 6,000 daltons. The amino acid sequence of said substance is in complete agreement with a part of the sequence predicted from the above-mentioned mRNA. It is therefore presumable that an hEGF precursor synthesized intracellularly and having a higher molecular weight than hEGF is secreted extracellularly and partly occurs as hEGF in urine and partly occurs as a partially processed precursor in urine. Said researchers obtained more or less pure HMW-hEGF from a urine component adsorbed on Celite by using DEAE-cellulose, CM-cellulose, Sephacryl S-200, reversed phase HPLC (high-performance liquid chromatography) technique, etc. The yield was about 3%. Tsukamoto et al. obtained HMW-hEGF in 1.4% yield by gel filtration with Bio-Rex 70 ion exchanger, anti-hEGF-Sepharose, Sephadex G-50 and Sephacryl S-200, further followed by reversed phase HPLC [Biochemical and Biophysical Research Communications, 145, 126-133 (1987)].

Findings of pathological importance have recently been obtained [Kurt Stromberg et al., Cancer Research, 47, 1190-1196 (1987)]. Thus, it is reported that HMW-hEGF is present in significantly high concentrations in urine samples from brain tumor patients and that tumor removal by surgery results in disappearance of HMW-hEGF from urine. Since this very interesting report, HMW-hEGF has acquired a deeper significance from a pathological and clinical standpoint.

However, the yield of HMW-hEGF, when it is extracted from urine, is very low, as can be seen in the above-cited report of Charles D. Mount et al., and this constitutes an obstacle to further advancement of the applicability study of that substance. In addition, it is difficult to assay HMW-hEGF by ordinary methods since by nature it immunologically crosses hEGF.

Accordingly, the advent of a simple method for accurate assay of said substance is awaited so that the relation between the occurrence of brain tumor and the body fluid concentration of said substance, for instance, can be further studied.

The present inventors separated and purified HMW-hEGF from a large quantity of urine by their original method and produced anti-HMW-hEGF monoclonal antibody-producing hybridomas using the purification product as a material. Among about 1,000 hybridoma cell lines obtained, they could find several cell lines capable of reacting with HMW-hEGF alone and incapable of reacting with hEGF. Using these cell lines, they produced and purified anti-HMW-hEGF antibodies in large quantities. They made intensive investigations using them and, as a result, succeeded in establishing two enzyme immunoassay methods by which HMW-hEGF can be assayed in a selective manner. Furthermore, they established a simple and timesaving method of producing HMW-hEGF from human urine by conducting affinity chromatography using carriers carrying said antibodies immobilized thereon.

The present invention, which is based on these novel findings, provides a monoclonal antibody which belongs to the class of immunoglobulins IgG and which recognizes the high-molecular-weight human epidermal growth factor having a molecular weight of about 33 kilodaltons but does not recognize the human epidermal growth factor having a molecular weight of about 6,000 daltons; a method of producing said monoclonal antibody which comprises culturing hybridomas produced by the fusion of myeloma cells with cells of a mammal immunized with the high-molecular-weight human epidermal growth factor having a molecular weight of about 33 kilodaltons as an antigen and recovering the anti-high-molecular-weight human epidermal growth monoclonal antibody; a method for enzyme immunoassay of the high-molecular-weight human epidermal growth factor which comprises immobilizing human epidermal growth factor species including the high-molecular-weight one as contained in a sample, reacting the above-mentioned monoclonal antibody with the immobilized human epidermal growth factor species and measuring the quantity of the antibody that has reacted by an enzyme immunoassay technique; a method of producing the high-molecular-weight human epidermal growth factor which comprises applying a body fluid containing said high-molecular-weight human epidermal growth factor to a column of a packing with the above-mentioned monoclonal antibody immobilized thereon to thereby cause said growth factor to be adsorbed on said packing and then eluting said growth factor from the column; and a strain of hybridomas produced by the fusion of myeloma cells with cells of a mammal immunized with the high-molecular-weight human epidermal growth factor, said strain being capable of producing the above-mentioned monoclonal antibody.

While HMW-hEGF, which is required for the production of the monoclonal antibody and hybridoma strain according to the invention, can be obtained by any known method, the present inventors prepared HMW-hEGF in about 7% yield (the total quantity of h-EGF plus HMW-hEGF in starting urine material being taken as 100%) by salting out human urine with ammonium sulfate and then carring out ion exchange chromatography on Amberlite IRA-93 and DEAE-Sephadex A-25, gel filtration on Bio-Gelp-10, affinity chromatography on anti-EGF-Sepharose and gel filtration on Sephadex G-100, in that order.

A hybridoma strain capable of producing a monoclonal antibody to HMW-hEGF can be produced, for example, by immunizing an animal, such as an Balb/C mouse, by a conventional method with the HMW-hEGF obtained as described above, excising the spleen from the immunized animal, purifying lymphocytes from said spleen and causing fusion of said lymphocytes with myeloma cells cultured in advance. The fusion can be carried out by the polyethylene glycol method or electroporation method, for instance. Desired hybridoma cells can be selected using HAT medium suited for utilization of aminopterine resistance. Screening for antibody-producing hybridomas can be performed by enzyme immunoassay using the HMW-hEGF obtained from human urine. For such screening, other immunological methods are also usable, for example radioimmunoassay, radioreceptor assay, radioimmunoprecipitation, Western blotting, etc.

The present inventors have established four cell lines capable of producing a monoclonal antibody specific to HMW-hEGF from among about 1,000 hybridoma strains by the screening method mentioned above. One of the four cell lines has been deposited with the Fermentation Research Institute, Agency of Industrial Science and Technology, under Budapest Treaty (Accession No. FERM BP-2573). In addition, the inventors have obtained two cell lines capable of producing an antibody which recognizes HMW-hEGF and hEGF. These six cell lines each can be readily grown in the murine peritoneal cavity as well to give an antibody in large quantities.

These antibodies can be purified to a high level of purity by any of well-known purification means, for example salting out, ion exchange, affinity chromatography using protein A, chromatography using hydroxyapatite, etc. The antibodies or Fab′ fragments derived therefrom by digestion with pepsin can be used for constructing ELISA (enzyme-linked immunosorbent assay) systems. On that occasion, enzyme-labeled antibodies obtained by linking alkaline phosphatase, peroxidase or the like directly to the antibodies may be used, or an enzyme-labeled anti-mouse IgG antibody or the like may be used as a secondary antibody. By appropriately combining such techniques, HMW-hEGF in body fluids can be specifically assayed using a monoclonal antibody prepared from the culture supernatant obtained by culturing the above-mentioned HMW-hEGF-specific monoclonal antibody-producer, without crossing by hEGF. It is also possible to establish an assay system using the Western blotting technique [Harry Towbin et al., Proc. Natl. Acad. Sci. USA, 76, 4350-4354 (1978)] by using the above-mentioned monoclonal antibody. This HMW-hEGF assay method is another very useful contribution to clinical diagnosis.

Thus, a body fluid sample from a patient is electrophoresed, proteins are transferred to an appropriate support, for example a nitrocellulose membrane, by heat treatment or electrically, portions of the membrane where no proteins have been adsorbed are blocked with skimmed milk-containing PBS(-) (divalent ion-free phosphate-buffered saline), and the membrane is reacted with an anti-HMW-hEGF antibody. If present, HMW-hEGF on the membrane can be detected using an enzyme-labeled anti-mouse IgG or ¹²⁵I-labeled protein A, for instance.

It is a more useful result that an affinity chromatography column can be prepared by immobilizing the above-mentioned monoclonal antibody on a carrier, such as Sepharose. The use of such column makes it possible to separate and purify HMW-hEGF from human urine in one step and in high yields. To the contrary, the prior art methods are complicated and give only very low yields.

### Reference Example

HMW-hEGF was extracted and purified as reported by the present inventors at the Annual Meeting of the Japanese Biochemical Society for 1986, namely as follows. Fresh human urine was salted out with ammonium sulfate, then purified on the ion exchange resins Amberlite IRA-93 (Japan Organo, Tokyo) and DEAE-Sephadex (Pharmacia Japan, Tokyo), and further subjected to gel filtration on Bio Gel P-10 (Bio-Rad, USA). A fraction showing EGF activity was purified by affinity chromatography using Sepharose 4B (Pharmacia Japan, Tokyo) with a polyclonal antibody* immobilized thereon. The eluate was subjected to gel filtration on Sephadex G-100 (Pharmacia Japan, Tokyo) to give a high-molecular-weight fraction showing hEGF activity. The hEGF activity was assayed by radioreceptor assay using HeLa cells. The hEGF activity recovered as the high-molecular-weight fraction amounted to 6-8% with the hEGF activity in urine being taken as 100%. Analysis of said fraction by SDS-electrophoresis revealed that the purity of HMW-hEGF was not less than 90%.
* An antiserum obtained by immunization of a rabbit with hEGF as an antigen was purified on protein A-Sepharose (Pharmacia Japan, Tokyo), followed by immobilization of the antibody thus purified.

### Example 1

### Hybridoma production

An emulsion containing 17.5 µg of the HMW-hEGF obtained in the Reference Example in complete Freund's adjuvant (Difco Laboratories, USA) was subcutaneously administered to Balb/C mice (obtained from Shizuoka Laboratory Animal Center, Shizuoka, Japan). Two weeks later, and four weeks later, the same dose of HMW-hEGF emulsified in incomplete Freund's adjuvant (Difco Laboratories, USA) was intravenously administered to the animals. After a further two weeks, HMW-hEGF was administered in the same manner but without any adjuvant. Three days thereafter, the spleen was excised, washed with divalent ion-free phosphate-buffered saline according to Dulbecco ["PBS(-)"] and minced therein to give a splenocyte suspension. P3x63Ag8655 mouse myeloma cells, which were a gift of Dr. J. F. Kearney at Alabama University, were used for fusion with the splenocytes. This cell line is a Balb/C mouse-derived one quite lacking in the ability to synthesize antibody-related proteins, hence incapable of secreting any immunoglobulin; fusion products alone are capable of antibody secretion. The myeloma cells were grown in RPMI 1640 medium (Nissui Pharmaceutical, Tokyo) supplemented with 10% fetal calf serum (Dainippon Pharmaceutical, Tokyo) and 10⁸ myeloma cells were recovered by 5-minute centrifugation at 1,500 rpm and then mixed with splenocytes prepared from two mice in the above manner. The mixed cell suspension was centrifuged at 1,500 rpm for 5 minutes and the cells were resuspended in PBS(-). The cells were repeatedly washed by repeating the above procedure. To the thus-washed cells was added dropwise 1.5 ml of a two-fold dilution of PEG-4000 (Sigma Chemical, USA) in PBS(-). After allowing the mixture to stand for 1 minute, 40 ml of serum-free RPMI 1640 medium was added dropwise and, immediately after completion of the dropping, the mixture was centrifuged at 1,500 rpm for 5 minutes. The fused cell sediment was suspended in HAT medium* and the suspension was distributed in 100-µl portions into wells of nine 96-well microplates (Corning, USA). The plates were incubated at 37°C for 2 to 3 weeks in an incubator where the carbon dioxide concentration was maintained at 5%. Hybridomas produced by the fusion of splenocytes with myeloma cells and capable of growing in HAT medium were selected.
* Prepared by adding 100 µM hypoxanthine, 0.1 µM aminopterine and 1.6 µM thymidine (all obtained from Sigma Chemical, USA) to RPMI 1640 medium supplemented with 10% fetal calf serum.

### Example 2

### Screening for antibody-producing hybridoma strains

A nitrocellulose membrane (0.45 µM; Bio-Rad, USA) was fixed on each 96-well manifold ("Bio-Dot"; Bio-Rad, USA) and HMW-hEGF was caused to be adsorbed on the membrane portion on each well in an amount equivalent to 70 ng of HMW-hEGF per well by allowing an HMW-hEGF solution to move on the membrane in the manner of spontaneous falling. For blocking those portions of the membrane where adsorption had not occurred, 250 µl of PBS containing 1% bovine serum albumin (Sigma Chemical, USA) was added to each well. Each well was then washed with PBS(-) containing 0.05% Tween 20 [TPBS(-)] by the aspiration method. The culture supernatant on each of the wells of the microplate where hybridoma cell growth in HAT medium was observed was transferred, in an amount of 100 µl, to a well of the manifold and allowed to react with HMW-hEGF for 1 hour. Then, the manifold was washed with TPBS. A solution prepared by diluting alkaline phosphatase-labeled anti-mouse IgG (Promega, USA) 7,000-fold with PBS(-) containing 1% bovine serum albumin was distributed in 100-µl portions into the wells and the reaction was allowed to proceed. After washing the manifold with TPBS, a substrate solution, namely Tris hydrochloride buffer (pH 9) containing 1 mM magnesium chloride, 30 mg/dl nitro blue tetrazolium and 15 mg/dl bromochloroindolyl phosphate, was distributed dropwise in 100-µl portions into the wells for color development. Hybridoma strains corresponding to the wells where a deep-blue pigment had deposited were selected as antibody-producing strains. In this way, about 300 antibody-producing strains were obtained.

### Example 3

### Determinations of specificities of antibodies produced by hybridomas

The specificity of the antibody produced by each of the strains selected in Example 2 was determined by the radioimmunoprecipitation method. Thus, a solution of hEGF purified by the conventional method and a solution of HMW-hEGF, each having a concentration of 1 mg/ml, were thoroughly dialyzed against 0.1 M phosphate buffer (pH 7.6) and then subjected to iodination using Bolton-Hunter reagent (Amersham, Great Britain). This reagent is known to permit iodination of proteins under very mild conditions while allowing the antigenicity to be maintained even after iodination. The Bolton-Hunter reagent in V-bottomed vials (250 µl per vial) was dried using dried nitrogen gas. Thereto was added 5 µl of the hEGF or HMW-hEGF solution.

After overnight reaction at 4°C, the unreacted Bolton-Hunter reagent and free iodine were removed from the iodinated hEGF by gel filtration using a column packed with 10 ml of Sephadex G-25 (Pharmacia Japan, Tokyo). In carrying out the gel filtration, 0.2% gelatin-containing PBS(-) was used so that nonspecific adsorption can be avoided. The two kinds of iodine-labeled hEGF were mixed together each in a concentration of 20,000 cpm to 200,000 cpm and the mixture was reacted overnight at 4°C with the supernatant from each cell strain confirmed in Example 2 to be an antibody producer. The reaction was carried out in a 1.5-ml Eppendorf centrifuge tube. Then, 5 µl of rabbit anti-mouse IgG (Cappel, USA) and 50 µl of Panisorbin (Calbiochem, USA) were added to the tube and the reaction was allowed to proceed for 1 hour. The subsequent centrifugation on a microcentrifuge gave a sediment. The sediment was washed four times with PBS(-) containing 0.1% Nonidet P-40 (nonionic surfactant; Sigma chemical, USA) and then treated with a loading buffer, namely 20 mM Tris hydrochloride buffer (pH 7.0) containing 2% sodium dodecyl sulfate (SDS; Bio-Rad, USA), glycerol and a small amount of bromphenol blue (Bio-Rad, USA) for elution of the radioactive hEFG adsorbed on Panisorbin. The eluate was electrophoresed on a 15% SDS-polyacrylamide gel. After applying an electric current at 40 V for 12 hours, the gel was taken out and an X-ray film was exposed thereto for autoradiography. The specificity of the monoclonal antibody secreted by each cell strain was determined by judging, based on the mobility of the radioactive site, whether the antibody recognized HMW-hEGF (33 kilodaltons) or hEGF (6,000 daltons). In the manner mentioned above, it was found that the antibodies produced by the strains Nos. 16, 25, 57 and 112 are reactive with HMW-hEGF but unreactive with hEGF. Their immunoglobulin subtypes were determined using HyClone's typing kit (HyClone, USA) and found to be IgG1 in all the four cases.

Furthermore, it was found that the strains Nos. 143 and 296 are producers of antibodies recognizing both HMW-hEGF and hEGF.

### Example 4

### Enzyme immunoassay (ELISA) system for HMW-hEGF

An anti-hEGF antiserum obtained by immunizing New Zealand White rabbits (obtained from Shizuoka Laboratory Animal Center) with hEGF by a conventional method was purified by means of protein A-Sepharose (Pharmacia Japan, Tokyo).

The anti-HMW-hEGF monoclonal antibody produced by the strain No. 25 obtained in Example 3 and the antibody recognizing both hEGF and HMW-hEGF as produced by the strain No. 143 were purified to give the respective IgG species. Both the IgG species were of the subtype IgG1 and were further purified using Bio-Rad's MAPS kit, whereby 3 mg of purified IgG was obtained from 100 ml of the No. 25 strain culture supernatant and 1.2 mg of purified IgG from 100 ml of the No. 143 strain culture supernatant. A solid phase ELISA system was established using these antibodies.

Thus, purified rabbit anti-hEGF antibody was diluted to 500 µg/ml with PBS(-) and the dilution was distributed dropwise in 100-µl portions into wells of each 96-well microplate (Nunc, USA) and allowed to stand overnight at 4°C for adsorption of the antibody.

Those portions on the plate where adsorption had not occurred were blocked by adding 200 µl of PBS(-) containing 1% bovine serum albumin dropwise to each well, followed by 1-hour incubation at room temperature. The plate was washed thoroughly with TPBS(-), samples prepared by serial dilution of an hEGF solution and an HMW-hEGF solution, each having a known concentration, with PBS(-) containing 1% bovine serum albumin were respectively added to the wells, and the reaction was allowed to proceed for 1 hour. Then, the plate was washed thoroughly with TPBS. The IgG purified from the No. 25 or 143 strain culture supernatant was diluted to 100 µg/ml PBS(-) containing 1% bovine serum albumin and the dilution was distributed dropwise in 100-µl portions into the wells. After allowing the reaction to proceed at room temperature for 1 hour, the plate was washed with TPBS and, then, a 3,000-fold dilution of alkaline phosphatase-labeled anti-mouse IgG for enzyme-labeled immunoassay (Bio-Rad, USA) was distributed dropwise in 100-µl portions into the wells. For diluting the antibody, PBS(-) containing 1% bovine serum albumin was used. After allowing the reacticn to proceed for 1 hour, the plate was washed thoroughly with TPBS. A substrate solution was prepared by dissolving 2 tablets of p-nitrophenyl phosphate (Bio-Rad, USA) in 10 ml of diethanolamine buffer (Bio-Rad, USA). The solution was distributed dropwise in 100-µl portions into the wells of the plate. After a 30-minute period for color development, the color reaction was terminated by adding 100 µl of 0.5 N sodium hydroxide to each well. Color intensity measurement was performed using a Titertek Multiskan colorimeter (Flow Laboratories, USA) equipped with a 405 nm filter.

When the No. 25 strain-derived IgG was used in the above assay system, hEGF was not detected at all even at a concentration of 1 mg/ml but HMW-hEGF reacted and gave colors in a dose-dependent manner up to 1 mg/ml. When, the No. 143 strain-derived IgG was used, hEGF as well as HMW-hEGF could be detected, the detection limits being 1 µg/ml and 100 ng/ml, respectively.

### Example 5

### Enzyme immunoassay by Western blotting technique

To a mixture of 5 µl of a 50 µg/ml solution of HMW-hEGF and 5 µl of a 50 µg/ml solution of hEGF was added 10 µl of gel loading buffer (Example 3) containing 8-mercaptoethanol. The resultant mixture was treated on a water bath maintained at 100°C for 5 minutes and then electrophoresed on a 15% polyacrylamide gel in the presence of 0.1% SDS.

The proteins separated on the gel were transferred electricaly to a nitrocellulose membrane in a transblotting apparatus (Bio-Rad, USA).

The above procedure was performed in the cell of said apparatus using trisglycine buffer containing 20% methanol and supplying an electric current of 200 mA for 2 hours. After protein transfer, the samples on the membrane were treated for fixation in 6.2% glutaraldehyde (Wako Pure Chemical Industries, Osaka) for 1 hour so that they could be prevented from being detached from the membrane. The membrane was washed thoroughly with PBS(-) and then allowed to stand in PBS(-) containing 5% skimmed milk for 1 hour for blocking those portions in which no antibody had been adsorbed. Then, the membrane was reacted with each monoclonal antibody or an immunized mouse serum (positive control) at a concentration of 100 µg/ml. Each antibody was diluted with the above-mentioned blocking solution and the reaction was carried out overnight at room temperature. After reaction, the membrane was washed well with TPBS(-), then reacted with alkaline phosphatase-labeled anti-mouse IgG and further treated, in the same manner as in Example 2, with a substrate solution containing nitro blue tetrazolium and bromochloroindolyl phosphate for color development. The culture supernatants derived from the strains Nos. 25, 112, 143 and 296 and the immunized mouse serum gave positive results in this assay. The culture supernatants from the strains Nos. 25 and 112 recognized HMW-hEGF alone while the other culture supernatants and said serum recognized both hEGF and HMW-hEGF.

### Example 6

### Extraction and purification of HMW-hEGF by affinity chromatography using anti-HMW-hEGF monoclonal antibody

Anti-HMW-hEGF monoclonal antibody was purified from the strain No. 25 culture supernatant using Bio-Rad's MAPS kit. A 20-mg portion of the purified IgG thus obtained was coupled to 100 ml of cyanogen bromide-activated Sepharose (Pharmacia Japan, Tokyo) in the conventional manner. Free cyanogen bromide groups were blocked with 0.1 M glycine buffer (pH 8) and then this Sepharose modification was packed into a column and bufferized with PBS(-). Ten liters of fresh urine collected from human males was adjusted to pH 8.5, the insoluble matter was filtered off, and the urine was adjusted to pH 7.5 and applied to the above-mentioned column. After washing the column with 100 ml of PBS(-) and then with 100 ml of PBS(-) containing 1 M sodium chloride, HMW-hEGF adsorbed was eluted with 0.1 M aqueous citric acid solution (pH 2.0). The above procedure gave 50 µg of HMW-hEGF in one step. The purity as determined by SDS-electrophoresis was about 85%. The yield as estimated by the ELISA technique mentioned in Example 4 was 90%.

Elution with a chaotropic ion-containing aqueous solution, such as aqueous ammonia or an aqueous solution of thiocyanic acid, gave the same results as obtained by the above-mentioned elution with aqueous citric acid solution.

In accordance with the present invention, novel anti-HMW-hEGF antibody-producing cells can be produced. Thus it has become possible to produce anti-HMW-hEGF monoclonal antibody in large quantities. It has also become possible to recover HMW-hEGF from human urine in a simple manner and in high yields by using said monoclonal antibody. By the prior art methods, it is difficult to recover HMW-hEGF from urine. Furthermore, said antibody is very useful in specifically assaying HMW-hEGF in human body fluids. Timesaving monitoring of cancer, in particular brain tumor, is now possible by the assay method according to the invention which uses said antibody.

## Claims

1. A monoclonal antibody which belongs to the class of immunoglobulins IgG and which recognizes the high-molecular-weight human epidermal growth factor having a molecular weight of about 33 kilodaltons but does not recognize the human epidermal growth factor having a molecular weight of about 6,000 daltons.

2. A monoclonal antibody as claimed in Claim 1 as obtained from a culture of hybridomas produced by the fusion of myeloma cells with cells of a mammal immunized with said high-molecular-weight human epidermal growth factor as an antigen.

3. A monoclonal antibody as claimed in Claim 2, wherein the hybridoma is FERM BP-2573.

4. A method of producing a monoclonal antibody as defined in any one of Claims 1 to 3 which comprises culturing hybridomas produced by the fusion of myeloma cells with cells of a mammal immunized with the high-molecular-weight human epidermal growth factor having a molecular weight of about 33 kilodaltons as an antigen and recovering the anti-high-molecular-weight human epidermal growth monoclonal antibody.

5. A method for enzyme immunoassay of the high-molecular-weight human epidermal growth factor having a molecular weight of about 33 kilodaltons which comprises immobilizing human epidermal growth factor species including the high-molecular-weight one as contained in a sample, reacting an anti-high-molecular-weight human epidermal growth factor monoclonal antibody as defined in any one of Claims 1 to 3 with the immobilized human epidermal growth factor species and measuring the quantity of the antibody that has reacted by an enzyme immunoassay technique.

6. A method as claimed in Claim 5, wherein after the reaction of the monoclonal antibody, the growth factor-antibody complex is reacted with an enzyme-labeled anti-immunoglobulin antibody derived from the same mammal as that from which said antibody has been derived, followed by measurement of the quantity of the labeling enzyme that has been bound to said complex.

7. A method as claimed in Claim 5, wherein the monoclonal antibody is used in an enzyme-labeled form, followed by measurement of the quantity of the labeling enzyme that has been bound.

8. A method of producing the high-molecular-weight human epidermal growth factor having a molecular weight of about 33 kilodaltons which comprises applying a body fluid containing said high-molecular-weight human epidermal growth factor to a column of a packing with a monoclonal antibody as defined in any of Claims 1 to 3 immobilized thereon to thereby cause said growth factor to be absorbed on said packing and then eluting said growth factor from the column.

9. A strain of hybridomas produced by the fusion of myeloma cells with cells of a mammal immunized with the high-molecular-weight human epidermal growth factor having a molecular weight of about 33 kilodaltons, said strain being capable of producing a monoclonal antibody as in Claim 2.

10. A strain as claimed in Claim 9, wherein said cells of a mammal are spleen cells and wherein said myeloma cells are incapable of secreting immunoglobulins.

11. The strain as claimed in Claim 9 deposited with the Fermentation Research Institute, Agency of Industrial Science and Technology under Accession No. FERM BP-2573 or a mutant or variant thereof.

## Patentansprüche

1. Monoklonaler Antikörper, der zur Klasse der Immunglobuline IgG gehört und den hochmolekularen humanen epidermalen Wachstumsfaktor mit einem Molekulargewicht von etwa 33 Kilodalton, nicht aber den humanen epidermalen Wachstumsfaktor mit einem Molekulargewicht von etwa 6000 Dalton erkennt.

2. Monoklonaler Antikörper nach Anspruch 1, wie er aus einer Kultur von Hybridzellen erhalten worden ist, die hergestellt wurden durch Fusion von Myelomzellen mit Zellen eines Säugers, welcher mit dem hochmolekularen humanen epidermalen Wachstumsfaktor als Antigen immunisiert worden ist.

3. Monoklonaler Antikörper nach Anspruch 2, bei dem die Hybridzelle FERM BP-2573 ist.

4. Verfahren zur Herstellung eines monoklonalen Antikörpers, wie er in einem der Ansprüche 1 bis 3 definiert ist, umfassend das Kultivieren von Hybridzellen, die durch Fusion von Myelomzellen mit Zellen eines Säugers hergestellt wurden, der mit dem hochmolekularen humanen epidermalen Wachstumsfaktor mit einem Molekulargewicht von etwa 33 Kilodalton als Antigen immunisiert wurde, und das Rückgewinnen des monoklonalen anti-hochmolekularen humanen epidermalen Wachstumsantikörpers.

5. Verfahren zum Enzym-Immunoassay des hochmolekularen humanen epidermalen Wachstumsfaktors mit einem Molekulargewicht von etwa 33 Kilodalton, umfassend das Immobilisieren humaner epidermaler Wachstumsfaktorspezies einschließlich der hochmolekularen als Bestandteil einer Probe, das Umsetzen eines monoklonalen anti-hochmolekularen humanen epidermalen Wachstumsfaktor-Antikörpers, wie er in einem der Ansprüche 1 bis 3 definiert ist, mit den immobilisierten humanen epidermalen Wachstumsfaktorspezies und das Messen der Quantität des Antikörpers, welche mittels einer Enzym-Immunoassay-Technik reagiert hat.

6. Verfahren nach Anspruch 5, bei dem der Wachstumsfaktor/Antikörper-Komplex nach der Umsetzung des monoklonalen Antikörpers mit einem Enzym-markierten Anti-Immunglobulin-Antikörper, welcher aus demselben Säuger stammt, aus dem der Antikörper abgeleitet worden ist, umgesetzt wird, gefolgt durch Messung der Quantität des markierenden Enzyms, die sich an den Komplex gebunden hat.

7. Verfahren nach Anspruch 5, bei dem der monoklonale Antikörper in einer Enzym-markierten Form verwendet wird, gefolgt durch Messung der Quantität des gebundenen markierenden Enzyms.

8. Verfahren zur Herstellung des hochmolekularen humanen epidermalen Wachstumsfaktors mit einem Molekulargewicht von etwa 33 Kilodalton, umfassend das Auftragen einer den hochmolekularen humanen epidermalen Wachstumsfaktor enthaltenden Körperflüssigkeit auf eine Säule mit einer Packung, in der ein monoklonaler Antikörper, wie er in einem der Ansprüche 1 bis 3 definiert ist, immobilisiert ist, um dadurch die Absorption des Wachstumsfaktors von der Packung herbeizuführen, und das anschließende Eluieren des Wachstumsfaktors aus der Säule.

9. Stamm von Hybridzellen, hergestellt durch Fusion von Myelomzellen mit Zellen eines Säugers, der mit dem hochmolekularen humanen epidermalen Wachstumsfaktor mit einem Molekulargewicht von etwa 33 Kilodalton immunisiert wurde, wobei der Stamm in der Lage ist, einen monoklonalen Antikörper gemäß Anspruch 2 zu bilden.

10. Stamm nach Anspruch 9, bei dem die Zellen eines Säugers Milzzellen sind, und bei dem die Myelomzellen nicht in der Lage sind, Immunglobuline auszuscheiden.

11. Stamm nach Anspruch 9, hinterlegt beim Fermentation Research Institute, Agency of Industrial Science and Technology, unter der Hinterlegungs-Nr. BP-2573 oder eine Mutante oder Variante davon.

## Revendications

1. Anticorps monoclonal qui appartient à la classe des immunoglobulines IgG et qui reconnait le facteur de croissance de l'épiderme humain de poids moléculaire élevé ayant un poids moléculaire d'environ 33 kilodaltons, nais qui ne reconnait pas le facteur de croissance de l'épiderme humain ayant un poids moléculaire d'environ 6 000 daltons.

2. Anticorps monoclonal selon la revendication 1, obtenu à partir d'une culture d'hybridomes produits par la fusion de cellules de myelome et de cellules d'un mammifère immunisé avec ledit facteur de croissance de l'épiderme humain de poids moléculaire élevé comme antigène.

3. Anticorps monoclonal selon la revendication 2, dans lequel l'hybridome est FERM BP-2573.

4. Procédé de préparation d'un anticorps monoclonal selon l'une quelconque des revendications 1 à 3, qui comprend la mise en culture d'hybridomes produits par la fusion de cellules de myelome et de cellules d'un mammifère immunisé avec le facteur de croissance de l'épiderme humain de poids moléculaire élevé ayant un poids moléculaire d'environ 33 kilodaltons comme antigène, et la récuperation de l'anticorps monoclonal anti-croissance de l'épiderme humain de poids moléculaire élevé.

5. Procédé pour l'essai immunologique enzymatique du facteur de croissance de l'épiderme humain de poids moléculaire élevé ayant un poids moléculaire d'environ 33 kilodaltons, qui comprend l'immobilisation d'espèces du facteur de croissance de l'épiderme humain contenant celui de poids moléculaire élevé tel que contenu dans un échantillon, la mise en réaction d'un anticorps monoclonal anti-facteur de croissance de l'épiderme humain de poids moléculaire élevé tel que défini dans l'une quelconque des revendications 1 à 3 avec les espèces du facteur de croissance de l'épiderme humain immobilisées, et la mesure de la quantité d'anticorps ayant réagi selon une technique d'essai immunologique enzymatique.

6. Procédé selon la revendication 5, dans lequel, après la réaction de l'anticorps monoclonal, le complexe facteur de croissance-anticorps est mis a réagir avec un anticorps anti-immunoglobuline marqué par une enzyme et dérivant du même mammifère que celui dont dérive ledit anticorps, puis on mesure la quantité de l'enzyme de marquage qui s'est liée audit complexe.

7. Procédé selon la revendication 5, dans lequel l'anticorps monoclonal est utilisé sous une forme marquée par une enzyme, puis on mesure la quantité d'enzyme de marquage qui s'est liée.

8. Procédé de préparation du facteur de croissance de l'épiderme humain de poids moléculaire élevé ayant un poids moléculaire d'environ 33 kilodaltons, qui comprend l'opération consistant à verser un fluide corporel contenant ledit facteur de croissance de l'épiderme humain de poids moléculaire élevé dans une colonne dont le garnissage comprend un anticorps monoclonal tel que défini dans l'une quelconque des revendications 1 à 3, qui y est immobilisé, pour provoquer ainsi l'absorption dudit facteur de croissance sur ledit garnissage, puis l'élution dudit facteur de croissance à travers la colonne.

9. Souche d'hybridomes produits par la fusion de cellules de myelome et de cellules d'un mammifère immunisé avec le facteur de croissance de l'épiderme humain de poids moléculaire élevé ayant un poids moléculaire d'environ 33 kilodaltons, ladite souche étant capable de produire un anticorps monoclonal selon la revendication 2.

10. Souche selon la revendication 9, dans laquelle lesdites cellules d'un mammifère sont des cellules de rate et lesdites cellules de myelome ne sont pas capables de sécréter des immunoglobulines.

11. Souche selon la revendication 9, déposée au Fermentation Research Institute, Agency of Industrial Science and Technology, sous le numéro d'entrée BP-2573, mutant ou variant de celle-ci.
